# EUROPEAN PATENT SPECIFICATION

(11) **EP 4 422 509 B1**
(45) Date of publication and mention of the grant of the patent: **25.11.2026**
(21) Application number: 22799982.8
(22) Date of filing: 18.10.2022
(51) Int. Cl.: A61B 10/00, A61B 5/097, A61B 5/08

(54) **SAMPLE COLLECTION SYSTEM AND ELUENT DELIVERY ELEMENT FOR THE SAME**
PROBENSAMMELSYSTEM UND ELUIERUNGSMITTELABGABEELEMENT DAFÜR
SYSTÈME DE COLLECTE D'ÉCHANTILLON ET ÉLÉMENT DE DISTRIBUTION D'ÉLUANT POUR CELUI-CI

(30) Priority: 29.10.2021 US 202163273300 P
(43) Date of publication of application: 04.09.2024
(73) Proprietor: 3M Innovative Properties Company, Saint Paul, Minnesota 55133-3427 (US)
(72) Inventor: SHERMAN, Audrey A., Saint Paul, Minnesota 55133-3427 (US); SITTER, Brett J., Saint Paul, Minnesota 55133-3427 (US); DOMBROWSKI, Alan R., Saint Paul, Minnesota 55133-3427 (US); STEPANOVA, Narina Y., Saint Paul, Minnesota 55133-3427 (US); BONILLA, Tonya D., Saint Paul, Minnesota 55133-3427 (US); BERRIGAN, Michael R., Saint Paul, Minnesota 55133-3427 (US); NERENG, Laura R., Saint Paul, Minnesota 55133-3427 (US)
(74) Representative: Vossius & Partner Patentanwälte Rechtsanwälte mbB
(86) International application number: PCT/IB2022/059989
(87) International publication number: WO 2023/073503

(56) References cited:
- WO-A1-2015/166246
- WO-A1-2022/172222
- WO-A1-2022/234044
- US-A- 5 174 959
- US-A1- 2015 377 748
- US-A1- 2021 059 560

## Description

### Field

The present disclosure relates to a sample collection device and system. The present disclosure relates to a bioaerosol collection device and system. The present disclosure further relates to an eluent delivery element for a sample collection device and system.

### Background

Diagnostic tests used to test for the presence of a virus or other pathogen in the airways, throat, or nasopharynx typically involve the insertion of a swab into the back of the nasal passage, the mid-turbinate area of the nasal passage, the anterior nares, or the throat to obtain a sample. The swab is then inserted into a container and analyzed or sent to a lab for processing. Other diagnostic tests involve collecting a saliva sample and then placing it in a container.

Recently, an unprecedented need for rapid viral testing has arisen due to the COVID-19 pandemic. Attempts to control the pandemic require a massive expansion of testing for SARS-CoV-2 virus in several different clinical and epidemiological contexts. Until recently, nasopharyngeal (NP) swabs were the United States Centers for Disease Control and Prevention's (CDC) preferred specimen type, as these specimens were thought to provide the most robust detection of patient infection. However, there are conflicting reports as to which of several specimen types bear the highest viral load.

Sensitivity is a complex issue, however, as detection in the upper airways (nasopharynx and oropharynx) is affected by multiple factors including duration of illness prior to testing, as well as the limit of detection ( LoD) of the RT-PCR assay used. Availability of NP swabs and the resources to establish NP collection sites with specimen collection personnel have remained critical bottlenecks. To resolve these issues, healthcare systems have adopted multiple different strategies, including engaging industrial manufacturers to mass produce novel 3D-printed NP swabs, as well as evaluating different specimen types and alternative sample-collection strategies, such as saliva.

Assessment of nasal swabs and saliva is a rapidly growing area of interest, specifically because these specimen sample type involves a less invasive procedure than NP swabs. Accordingly, such samples can be self-collected by patients with a simple set of instructions, alleviating the need for highly trained medical personnel for specimen collection and reducing use of personal protective equipment (PPE) in short supply.

Many of the US Food and Drug Administration Emergency Use Authorization (FDA EUA) RT-PCR assays have approval for use of nasal swabs as a specimen type as well as saliva, but how well these samples perform compared to NP swabs remains unclear. To date, nasal-swab studies have shown conflicting results, with some researchers reporting similar test performance to NP swabs and others finding decreased sensitivity.

Currently available at-home viral tests (e.g., COVID-19 tests) involve a nasal swab and a test kit (for example, the Ellume^{™} test, the Abbot^{™} BinaxNOW^{™} test, and the Lucira^{™} All-in-One test kit). Tests that utilize nasal swab samples or saliva contend with contaminants that can interfere with the various diagnostic tests. As a result, these sample types require a purification step when using RT-PCR molecular testing.

The need exists for a simpler and cleaner sample collection system and easy elution of samples in a simple-to-use procedure. Further, a need exists for a sample collection system that can also capture and elute samples that have a low SARS-CoV-2 viral load but are still capable of transmitting the virus to others. There is also a need for a more precise system to reduce human error and provide more repeatable a reliable test results.

US 5,174,959 discloses a breath component monitoring device comprising a blow tube and a disposable analyzer column containing a breakable ampule, wherein a breaker button located on a portable housing is manually depressed to break the ampule and release a reactant solvent into a reaction zone.

US 2015/0377748 A1 describes a breath analysis system utilizing a breath analysis cartridge for sample collection and a separate fluorescence analysis cartridge, wherein the fluorescence analysis cartridge includes a piercing member arranged to pierce a breakable barrier of an ampule assembly disposed within the breath analysis cartridge upon connection of the two cartridges.

### Summary

There is a need for an inexpensive, simple to use, and reliable sample collection system that may be used by laypeople to obtain a sample for testing for the presence of a target virus, target pathogen, or other target analyte, in a collected sample. There is a need for an inexpensive, simple to use, and reliable eluent delivery element that may be used by laypeople to transfer a collected sample from a sample collection device to an assay.

The present invention is defined by the independent claims. The sample collection system of the present disclosure includes a sample collection device for collecting a sample from exhalation airflow onto sample collection media and an eluent delivery element for eluting the sample onto an assay, such as a lateral flow assay, which may be used to analyze the sample.

It is desirable to provide a sample collection device and system that are easy to use. The device and system may advantageously be self-contained and optionally sterile. A self-contained (and optionally sterile) device and system may improve accuracy and reliability of pathogen testing due to the reduced contamination and background noise, unlike swabs and other test collection devices which may be contaminated upon use and/or during testing.

It is further desirable to provide a system which, after sample collection, provides for easy elution and transfer of the collected sample onto an assay for testing safely and without contamination. It is further desirable to provide a system that provides a metered dose of an eluent for easy elution and transfer of the collected sample onto an assay. A metered dose may improve accuracy of the analysis. According to embodiments disclosed herein, such systems are provided.

### Brief Description of Figures

FIG. 1A is a perspective view of a sample collection system according to an embodiment.
FIG. 1B is a cut-off perspective view of the sample collection system of FIG. 1A.
FIG. 2 is a schematic perspective view of a piercing element and sample collector of the system of FIG. 1A according to an embodiment.
FIGS. 3A-3C are schematics of the insertion of an eluent delivery element into the mouthpiece of the device of FIG. 1A according to an embodiment.
FIG. 4 is a schematic cross-sectional view of the mouthpiece and an eluent delivery element of the system of FIG. 1A in a coupled position according to an embodiment.
FIG. 5A is a cross-sectional view of a hollow needle piercing element for the system of FIG. 1A according to an embodiment.
FIG. 5B is a cross-sectional view of a blade-shaped piercing element for the system of FIG. 1A according to an embodiment.
FIG. 5C is a schematic perspective view of an arrow-head-shaped piercing element and mouthpiece for the system of FIG. 1A according to an embodiment.
FIG. 6 is a schematic cross-sectional side view of the system of FIG. 1A with an alternative piercing element according to an embodiment.
FIG. 7 is a schematic cross-sectional side view of the system of FIG. 1A with an alternative piercing element according to an embodiment.
FIG. 8 is a schematic cross-sectional side view of the system of FIG. 1A with an alternative piercing element according to an embodiment.
FIG. 9 is a schematic cross-sectional view of a sample collection system with an alternative piercing element according to an embodiment.
FIG. 10A is a schematic perspective view of an eluent delivery element of the system of FIG. 1A according to an embodiment.
FIG. 10B is a schematic bottom view of the eluent delivery element of FIG. 10A according to an embodiment.
FIG. 11 is a schematic cross-sectional side view of a mouthpiece for the system of FIG. 1A according to an embodiment.
FIG. 12A is a side view of a mouthpiece for the system of FIG. 1A according to an embodiment.
FIG. 12B is another side view of the mouthpiece of FIG. 12A.
FIG. 12C is a cross-sectional side view of the mouthpiece of FIG. 12A.
FIG. 12D is a top view of the mouthpiece of FIG. 12A.
FIG. 13A is a side view of an inlet part of the mouthpiece of FIG. 12A.
FIG. 13B is a cross-sectional side view of the inlet part of FIG. 13A.
FIG. 13C is a top view of the inlet part of FIG. 13A.
FIG. 13D is a perspective view of the inlet part of FIG. 13A.
FIG. 14A is a side view of a base of the mouthpiece of FIG. 12A.
FIG. 14B is a cross-sectional side view of the base of FIG. 14A.
FIG. 14C top view of the base of FIG. 14A.
FIG. 14D is a bottom view of the base of FIG. 14A.
FIG. 14E is a perspective view of the base of FIG. 14A.
FIG. 15A is a side view of a mouthpiece for the system of FIG. 1A according to an embodiment.
FIG. 15B is another side view of the mouthpiece of FIG. 15A.
FIG. 15C is a cross-sectional side view of the mouthpiece of FIG. 15A.
FIG. 15D is a top view of the mouthpiece of FIG. 15A.
FIG. 16A is a side view of an inlet part of the mouthpiece of FIG. 15A.
FIG. 16B is a cross-sectional side view of the inlet part of FIG. 16A.
FIG. 16C is a top view of the inlet part of FIG. 16A.
FIG. 16D is a perspective view of the inlet part of FIG. 16A.
FIG. 17A is a side view of a mouthpiece for the system of FIG. 1A according to an alternative embodiment.
FIG. 17B is a cross-sectional side view of the mouthpiece of FIG. 17A.
FIG. 17C top view of the mouthpiece of FIG. 17A.
FIG. 17D is a perspective view of the mouthpiece of FIG. 17A.
FIG. 17E is another perspective view of the mouthpiece of FIG. 17A.
FIG. 18A is a cross-sectional side view of a mouthpiece for the system of FIG. 1A according to an alternative embodiment.
FIG. 18B is a cross-sectional top view of an eluent delivery element for use with the mouthpiece of FIG. 18A.
FIG. 18C is a cross-sectional side view of the eluent delivery element of FIG. 18B.

### Definitions

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, the terms "polymer" and "polymeric material" include, but are not limited to, organic homopolymers, copolymers, such as for example, block, graft, random and alternating copolymers, terpolymers, etc., and blends and modifications thereof. Furthermore, unless otherwise specifically limited, the term "polymer" shall include all possible geometrical configurations of the material. These configurations include, but are not limited to, isotactic, syndiotactic, and atactic symmetries.

The terms "downstream" and "upstream" refer to a relative position based on a direction of exhalation airflow through the device. For example, the upstream-most element of the device is the inlet or mouthpiece element, and the downstream-most element of the device is the outlet or sample receiving area of the assay.

All headings provided herein are for the convenience of the reader and should not be used to limit the meaning of any text that follows the heading, unless so specified.

The term "i.e." is used here as an abbreviation for the Latin phrase id est, and means "that is," while "e.g." is used as an abbreviation for the Latin phrase exempli gratia and means "for example."

All scientific and technical terms used herein have meanings commonly used in the art unless otherwise specified. The definitions provided herein are to facilitate understanding of certain terms used frequently herein and are not meant to limit the scope of the present disclosure.

The term "about" is used here in conjunction with numeric values to include normal variations in measurements as expected by persons skilled in the art and is understood have the same meaning as "approximately" and to cover a typical margin of error, such as ±5 % of the stated value. Moreover, unless otherwise indicated, all numbers expressing quantities, and all terms expressing direction/orientation (e.g., vertical, horizontal, parallel, perpendicular, etc.) in the specification and claims are to be understood as being modified in all instances by the term "about."

Terms such as "a," "an," and "the" are not intended to refer to only a singular entity but include the general class of which a specific example may be used for illustration.

The terms "a," "an," and "the" are used interchangeably with the term "at least one." The phrases "at least one of" and "comprises at least one of" followed by a list refers to any one of the items in the list and any combination of two or more items in the list.

As used here, the term "or" is generally employed in its usual sense including "and/or" unless the content clearly dictates otherwise. The term "and/or" means one or all of the listed elements or a combination of any two or more of the listed elements.

The recitations of numerical ranges by endpoints include all numbers subsumed within that range (e.g., 1 to 5 includes 1, 1.5, 2, 2.75, 3, 3.80, 4, 5, etc. or 10 or less includes 10, 9.4, 7.6, 5, 4.3, 2.9, 1.62, 0.3, etc.). Where a range of values is "up to" or "at least" a particular value, that value is included within the range.

As used here, "have", "having", "include", "including", "comprise", "comprising" or the like are used in their open-ended sense, and generally mean "including, but not limited to." It will be understood that "consisting essentially of," "consisting of," and the like are subsumed in "comprising" and the like. As used herein, "consisting essentially of," as it relates to a composition, product, method or the like, means that the components of the composition, product, method or the like are limited to the enumerated components and any other components that do not materially affect the basic and novel characteristic(s) of the composition, product, method or the like.

The term "substantially" as used here has the same meaning as "significantly," and can be understood to modify the term that follows by at least about 90 %, at least about 95 %, or at least about 98 %. The term "not substantially" as used here has the same meaning as "not significantly," and can be understood to have the inverse meaning of "substantially," i.e., modifying the term that follows by not more than 10 %, not more than 5 %, or not more than 2 %.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

Any direction referred to here, such as "front," "back," "top," "bottom," "left," "right," "upper," "lower," and other directions and orientations are described herein for clarity in reference to the figures and are not to be limiting of an actual device or system or use of the device or system. Devices or systems as described herein may be used in a number of directions and orientations.

Any direction referred to here, such as "top," "bottom," "left," "right," "upper," "lower," and other directions and orientations are described herein for clarity in reference to the figures and are not to be limiting of an actual device or system or use of the device or system. Devices or systems as described herein may be used in a number of directions and orientations.

### Detailed Description

The present disclosure relates to a sample collection device and system. The present disclosure relates to a bioaerosol sample collection device and system.

The sample collection device includes sample collector that includes porous sample collection media along an airflow path formed by the device housing. The porous sample collection media is constructed to capture viruses, pathogens, or other analytes, carried in an exhalation airflow. The porous sample collection media may be a nonwoven material capable of capturing pathogens, viruses, or other analytes. After loading a sample onto the porous sample collection media, the sample may be eluted from the sample collection media onto an assay. The sample may be analyzed for the presence of a pathogen or other analyte of interest. According to an embodiment, the sample may be eluted by passing a liquid (e.g., eluent) through the porous sample collection media to elute the sample, including pathogens, viruses, or other analytes, bound to the porous sample collection media, forming an eluate, and allowing the eluate to flow onto the assay. The eluate may then be analyzed using the assay.

According to an embodiment, the sample collection system includes a sample collection device and an eluent delivery element. The sample collection device includes a housing with an airflow path extending from a proximal end to a distal end. The proximal end forms an air inlet. The distal end may be at or adjacent a sample receiving area of the assay. A piercing element is arranged within the airflow path. The sample collection device further includes a sample collector that includes porous sample collection media arranged to occlude the airflow path. The eluent delivery element includes a reservoir containing an eluent and a membrane disposed at a coupling end of the reservoir to seal the reservoir. The coupling end is constructed to couple with the proximal end of the airflow path (e.g., the inlet end) such that the piercing element pierces the membrane and releases the eluent from the reservoir.

According to an embodiment, a user may exhale into the sample collection device and load the sample collector with a sample of the exhalation airflow to form a loaded porous sample collection media. The user may exhale through an air inlet in the sample collection device. The air inlet may be formed as a mouthpiece. Alternatively, the sample collection device may have a nose piece constructed for exhaling through the nose (e.g., through one or both nostrils). The user may exhale through an air inlet in the mouthpiece or the nose piece of the sample collection device. The housing is constructed so that by exhaling through the air inlet, the exhalation airflow passes through the porous sample collection media. The porous sample collection media is constructed to capture viruses, other pathogens, or other analytes, from the exhalation airflow. The user may then connect the eluent delivery element to the mouthpiece of the device to apply an eluent to the loaded porous sample collection media to elute the loaded sample onto an assay.

The sample collection system may include a lateral flow assay adjacent the porous sample collection media. In some embodiments, the porous sample collection media is in direct contact with the lateral flow assay. In other embodiments, the porous sample collection media is not in direct contact with the lateral flow assay.

The housing may include an inlet part or a mouthpiece defining the proximal end of the airflow path. The piercing element may be recessed in the inlet part or mouthpiece. The piercing element is constructed to pierce, puncture, score, cut, slit, or otherwise rupture the membrane of the eluent delivery reservoir and to create an opening in the membrane. These piercing functions are referred to here collectively as "piercing." The piercing element may remain inside the eluent delivery reservoir or may break off during or after piercing. Rotating the eluent delivery element as it is inserted in the airflow path may aid in the piercing of the membrane and may create an opening in the membrane that is larger than the size of the piercing element. The piercing element may further be constructed to act as a fluid guide. The piercing element may be constructed to draw fluid from the reservoir. The piercing element may be constructed to guide the flow of eluent onto the porous sample collection media and optionally onto a sample receiving area on the assay.

The piercing element may be recessed in the mouthpiece by any suitable distance that will allow the eluent delivery reservoir to be pierced yet reduces or minimizes the likelihood of a user inadvertently coming into contact with the piercing tip. A suitable recessed distance may be determined based on the particular dimensions of the mouthpiece and the eluent delivery element. The piercing element may be recessed, for example, by a distance of 4 mm or more, 5 mm or more, or 6 mm or more. The piercing element may be recessed by a distance of 10 mm or less, 9 mm or less, or 8 mm or less. In some embodiments, the piercing element is recessed by 4 mm to 10 mm or by 5 mm to 8 mm.

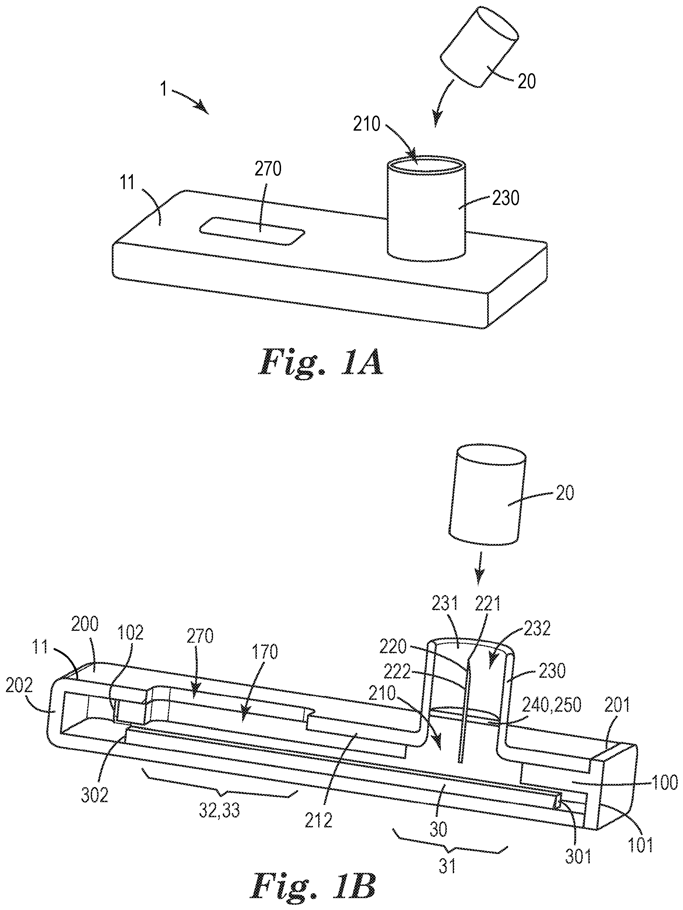

Referring now to FIGURES 1A and 1B, an exemplary embodiment of sample collection system 1 with a sample collection device 10 and eluent delivery element 20 is shown. The sample collection device 10 includes a housing 11 that houses the assay 30 (e.g., a lateral flow assay). A mouthpiece 230 extends from the housing 11, forming an airflow path 210. A sample collector 250 including porous sample collection media 252 occludes the airflow path 210. The mouthpiece 230 is constructed to receive an eluent delivery element 20. A piercing element 220 is disposed in the airflow path 210 and configured to pierce the eluent delivery element 20.

The construction of the sample collection device 10 is not particularly limited, as long as the sample collection device 10 facilitates the sample collector 250, airflow path 210, piercing element 220, eluent delivery element 20, and assay 30. The housing of a suitable sample collection device may be modified to include the mouthpiece 230, 430, 530, 530' with airflow path, sample collector, and piercing element of the present disclosure. Examples of such sample collection devices that may be modified include those described in U.S. Provisional Patent Application Nos. 63/200,058 (filed on February 12, 2021), 63/202,140 (filed on May 28, 2021), 63/200,958 (filed on April 6, 2021), 63/202,143 (filed on May 28, 2021), 63/201,983 (filed on May 21, 2021), 63/203,441 (filed on July 22, 2021), and 63/203,442 (filed on July 22, 2021).

The housing 11 of the sample collection device may be constructed from one or more pieces or parts. In some embodiments, as shown in FIGURES 1A and 1B, the housing 11 includes a first part 100 that carries the assay 30 (e.g., a lateral flow assay) and a second piece 200 that houses the first part 100 and assay 30 and provides or connects with the airflow path 210 and sample collector 250. The mouthpiece 230 may be a separate piece coupled with the housing 11 or may be integrally formed with the housing 11, as shown. The housing 11 may include a viewing window 270 that allows a user to view the result of the assay 30.

The mouthpiece 230 may define the proximal end 211, including the air inlet 231, of the airflow path 210. The mouthpiece 230 may include a support element 240 or support grid. The piercing element 220 element extends proximally (toward the air inlet 231) from the support element 240 or support grid. The piercing element 220 may be recessed in the mouthpiece 230 by a distance D220. The piercing element 220 may be recessed by a sufficient distance to reduce or minimize the likelihood of a user inadvertently coming into contact with the piercing tip 221 of the piercing element 220. The piercing element 220 is constructed to pierce, puncture, score, cut, slit, or otherwise rupture the membrane 23 of the eluent delivery element 20. The piercing element 220 may further be constructed to act as a fluid guide. The piercing element 220 may be constructed to draw fluid from the reservoir 21. The piercing element 220 may be constructed to guide the flow of eluent 22 onto the porous sample collection media 252 and optionally onto a sample receiving area 31 on the assay 30.

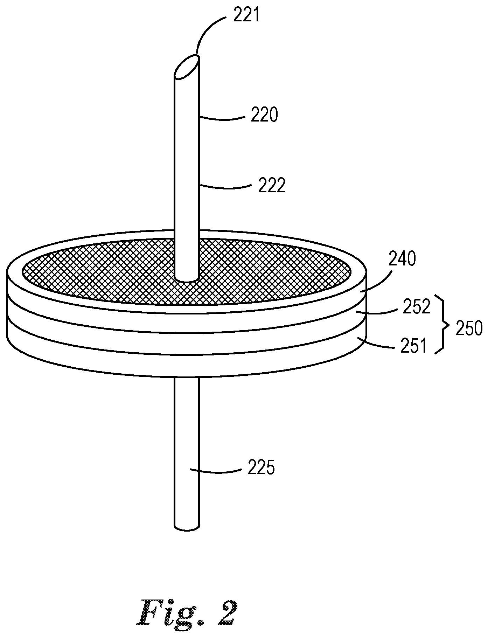

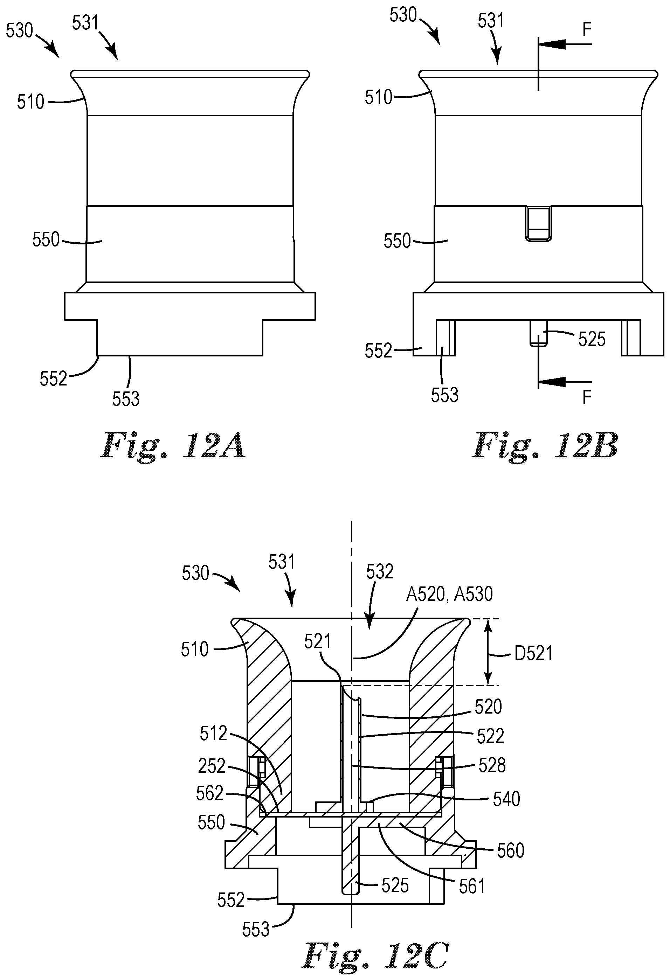

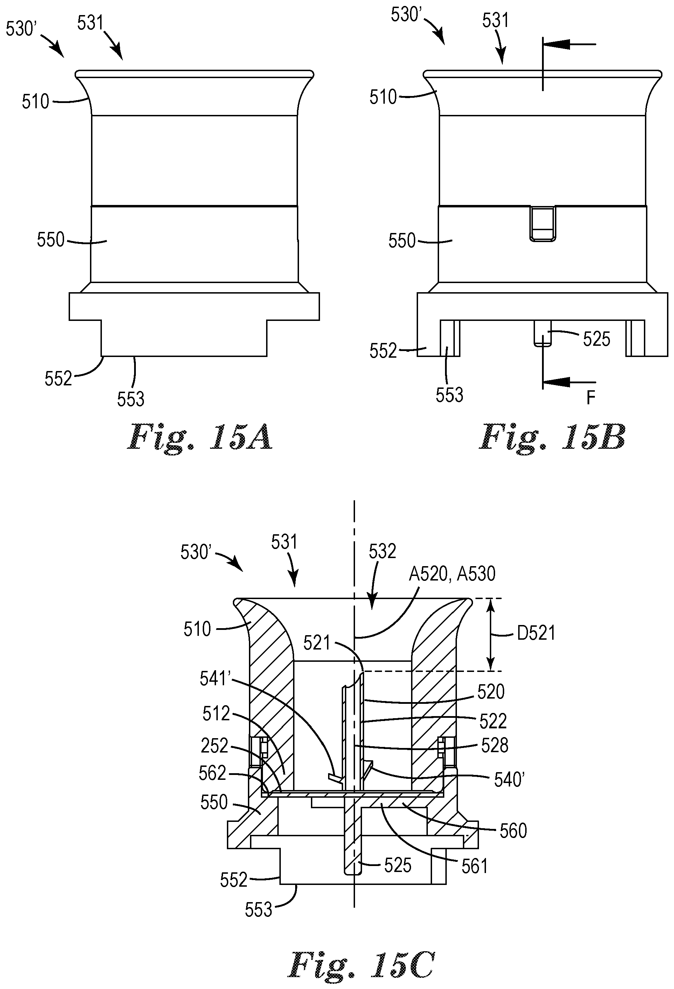

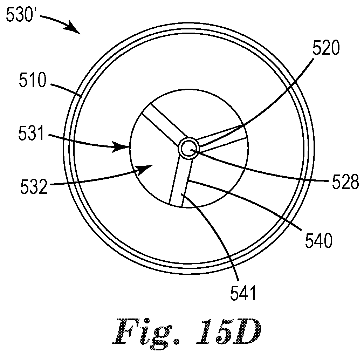

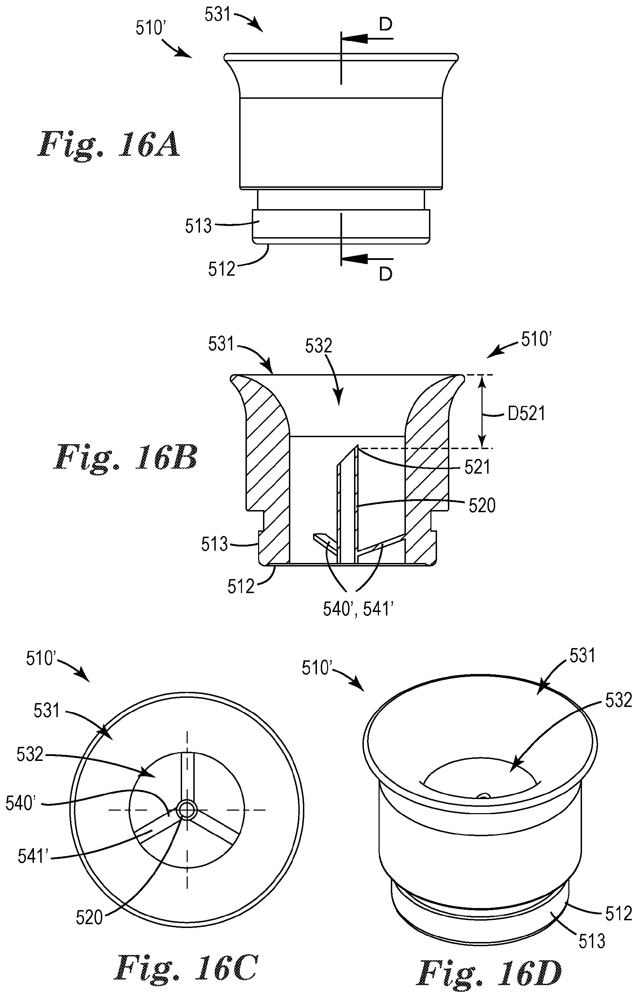

FIGURE 2 shows a schematic view of the piercing element 220, support element 240 or support grid, and sample collector 250. The piercing element 220 may have a shaft 222 that extends proximally from the support element 240 or support grid. The piercing element 220 may also extend distally (toward the assay 30) from the support element 240 and include a distal section 225. The support element 240 or support grid may serve multiple purposes, including supporting the piercing element 220 within the airflow path 210, but optionally also supporting the porous sample collection media 252. That is, the support element 240 or support grid may form part of the sample collector 250. In some embodiments, the support element 240 or support grid may support either the porous sample collection media 252, the piercing element 220, or both the porous sample collection media 252 and the piercing element 220. In some embodiments, the sample collector 250 includes a separate media support element 251. The porous sample collection media 252 may be disposed adjacent the support element 240 or a media support element 251 (if provided), or be disposed between the support element 240 and the media support element 251. In some embodiments, such as those shown in FIGURES 12A-16D, the support element 240 may be provided in an inlet part 510 of the mouthpiece 530 and the media support element 251 may be provided in a base 550 of the mouthpiece 530. The support element 240 or support grid may have any suitable construction. For example, the support element 240 or support grid may have a wheel-and-spoke design, a mesh, or any other suitable configuration. In some embodiments, the support element 240 or support grid may be tapered or angled toward the center of the airflow channel and toward the assay to facilitate fluid flow to the assay, as shown in FIGURES 15C, 15D, and 16B.

According to an embodiment, a user may exhale into the mouthpiece 230 of the sample collection device 10 and load the porous sample collection media 252 of the sample collector 250 with a sample of the exhalation airflow. The user may exhale through the air inlet 231 of the mouthpiece 230. The exhalation airflow passes along the airflow path 210 and through the porous sample collection media 252.

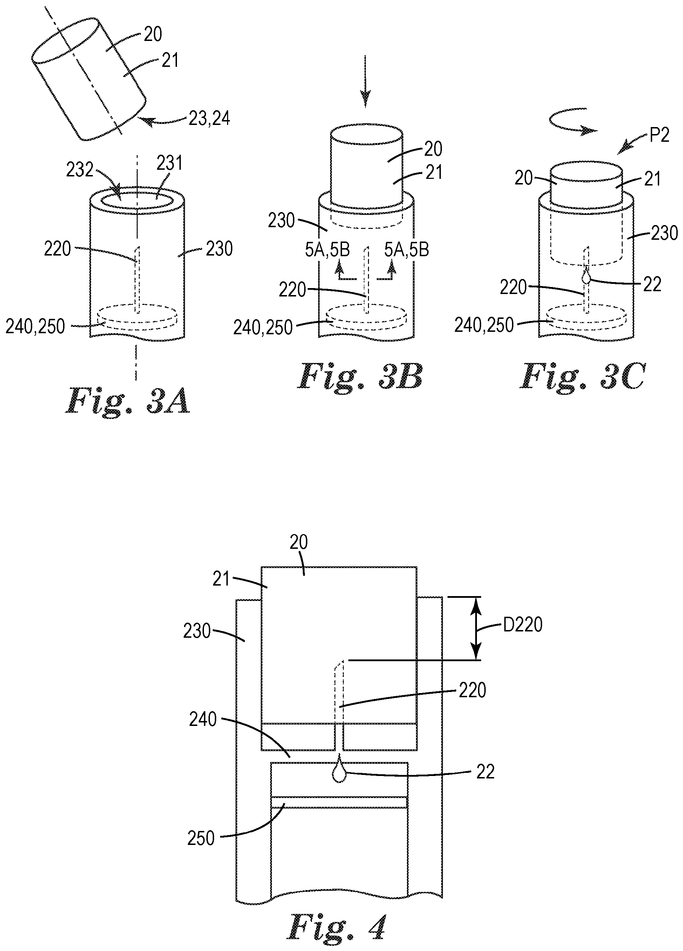

After loading the sample collector 250 (e.g., the porous sample collection media 252) with a sample, the user may connect the eluent delivery element 20 with the sample collection device 10 to apply the eluent 22 to the loaded media. The coupling of the eluent delivery element 20 with the sample collection device 10 is shown schematically in FIGURES 3A-3C. The sample collection system 1 has an uncoupled position P1 (FIGURE 3A) and a coupled position P2 (FIGURE 3C). In the coupled position P2, the eluent delivery element is coupled with the mouthpiece 230 of the sample collection device 10 and delivers eluent from the reservoir onto the porous sample collection media 252. The eluent delivery element 20 may be coupled with the sample collection device 10 by inserting the coupling end 24 of the eluent delivery element 20 into the air inlet 231 and the interior cavity 232 of the mouthpiece 230. The eluent delivery element 20 may be pushed, twisted, or snapped into place. The sample collection system 1 may have an indicator configured to indicate whether the sample collection system is in the coupled position P2. The indicator may be, for example, an audible indicator (e.g., a "click" sound) or a visual indicator (e.g., corresponding aligning features on the eluent delivery element and the sample collection device). When the eluent delivery element 20 is in the coupled position P2, the longitudinal center axis A20 of the eluent delivery element 20 may be coaxial with the longitudinal center axis A230 of the mouthpiece 230.

According to an embodiment, the eluent delivery element 20 is constructed to deliver a metered dose of the eluent 22 from the reservoir 21 onto the porous sample collection media 252 in the coupled position P2.

The sample collection system 1 may be constructed so that after the eluent delivery element 20 is inserted and pierced, the eluent delivery element 20 is partly retracted axially to facilitate eluent flow from the reservoir 21. The eluent delivery element 20 may have a piercing position, e.g., the coupled position P2, and a fluid delivery position, where the fluid delivery position is axially proximal (along the longitudinal axis A230 of the mouthpiece 230) to the piercing position P2.

FIGURE 4 shows a cross-sectional schematic of the eluent delivery element 20 in the coupled position P2 with the mouthpiece 230. In the coupled position P2, the piercing element 220 extends into the reservoir 21.

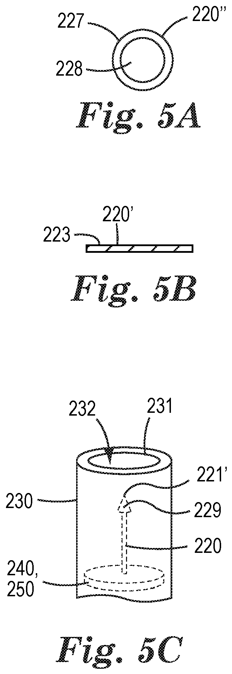

The piercing element 220 may have any suitable shape. Various shapes are shown in FIGURES 5A-5C. The piercing element 220 includes a tip 221 and a shaft 222. In some embodiments, the piercing element 220 is shaped like a needle 227 or a blade 223. A needle 227 shaped piercing element 220 may optionally have a hollow center 228, as shown in the cross-section in FIGURE 5A. The piercing element 220 may have non-circular cross-sectional shape along its longitudinal center axis, such as the cross-sectional shape of the blade 223 shown in FIGURE 5B. The cross-sectional shape or size of the piercing element 220 may vary along the length of the piercing element. That is, the piercing element 220 may have a first cross-sectional shape and a second cross-sectional shape along the longitudinal center axis A220, where the second cross-sectional shape is different from the first cross-sectional shape. The piercing element 220 may have a first cross-sectional diameter and a second cross-sectional diameter along the longitudinal center axis, where the second cross-sectional diameter is different from the first cross-sectional diameter. For example, the cross-sectional shape or size or both may be different at the tip 221 of the piercing element 220 than along the shaft 222. An example of a piercing element 220 that has a tip 221' with a different cross-sectional shape and size than the shaft 222 is the piercing element 220' with an arrow-head shaped tip 229 shown in FIGURE 5C. The arrow-head shaped tip 229 may have a largest cross-dimension at the base of the tip 229 that is greater than the cross-dimension of the shaft. Having a tip 221, 229 with a larger cross-dimension than the shaft 222 may help drain the reservoir even if the piercing element remains in the pierced opening of the membrane.

The mouthpiece 230 (and the airflow path 210) may define a longitudinal center axis A230. In some embodiments, the piercing element 220 extends parallel to the longitudinal center axis A230. In some embodiments, the piercing element 220 is not co-axial with the mouthpiece 230 and the airflow path 210. In some embodiments, the piercing element 220 is co-axial with the mouthpiece 230 and the airflow path 210.

The piercing element 220 may be constructed to act as a fluid guide and may include features that help draw fluid from the reservoir 21 or guide the flow of eluent 22 onto the porous sample collection media 252 and optionally onto a sample receiving area 31 on the assay 30. In some embodiments, the piercing element 220 is a hollow needle 227 having a hollow center 228 extending from an open first end to an opposing open second end. The piercing element 220 may have surface texture, surface treatment, or material configured to facilitate wicking or capillary flow. For example, the piercing element 220 may have surface texture, surface treatment, or material that helps to draw out fluid from the reservoir 21 or to flow the fluid toward the porous sample collection media 252 or both. Examples of materials that can facilitate wicking or capillary flow include porous materials, such as cellulosic materials, and hydrophilic materials, such as hydrophilic polymers. Examples of surface textures that can facilitate wicking or capillary flow include angled channels (e.g., channels having a V-shaped cross section), micro channels, capillary channels, and surface roughness. Examples of surface treatments that can facilitate flow of the eluent include corona treatment to render a surface easily wettable.

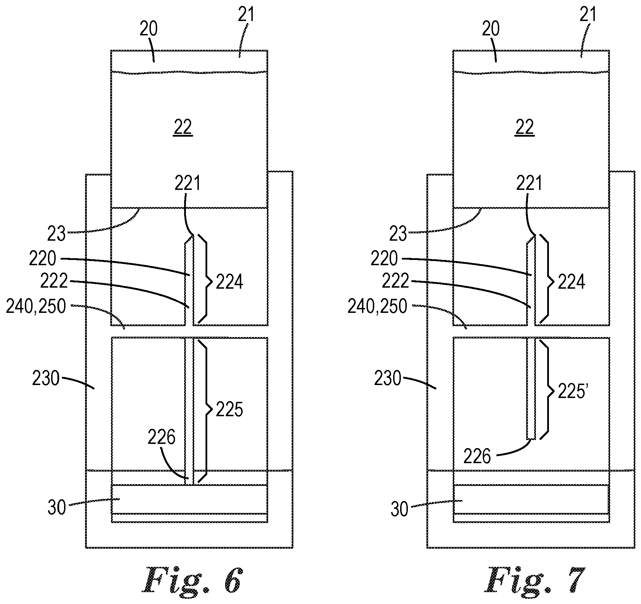

In some embodiments, the piercing element 220 includes a proximal section 224 (e.g., the section that functionally forms the piercing element 220) extending in a proximal direction from a support element 240 and a distal section 225 extending in a distal direction from the support element 240. Examples of such embodiments are shown in FIGURES 6 to 9.

The distal section 225 may be in direct contact with a sample receiving area 31 of the lateral flow assay 30, as shown in FIGURE 6. That is, the distal end 226 contacts the sample receiving area 31 of the lateral flow assay 30. In some embodiments, as shown in FIGURE 7, the distal section 225' is not in direct contact with the sample receiving area 31.

The distal section 225 (or the entire piercing element 220, including the proximal section 224 and the distal section 225) may have surface texture, surface treatment, or material configured to facilitate wicking or capillary flow. Examples of materials that can facilitate wicking or capillary flow include porous materials, such as cellulosic materials, and hydrophilic materials, such as hydrophilic polymers. Examples of surface textures that can facilitate wicking or capillary flow include angled channels (e.g., channels having a V-shaped cross section), micro channels, capillary channels, and surface roughness. Examples of surface treatments that can facilitate flow of the eluent include corona treatment to render a surface easily wettable.

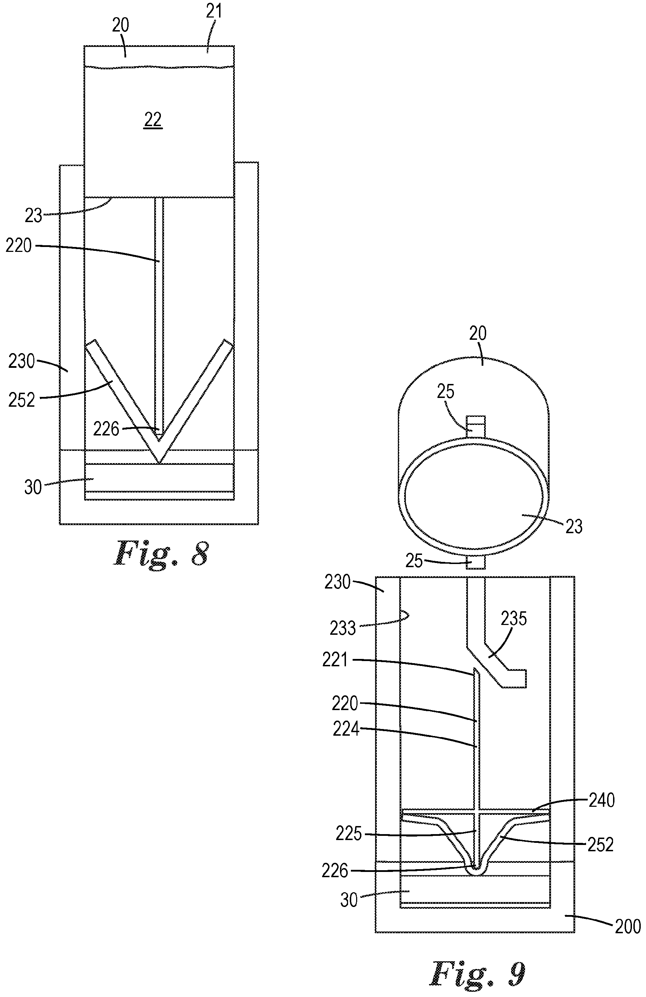

In some embodiments, shown in FIGURES 8 and 9, the porous sample collection media 252 is disposed below the distal section 225 of the piercing element 220, which extends from the support grid or support element 240 toward the assay 30 and pushes or deforms the porous sample collection media 252 into a non-planar shape, such as a cone or a cup shape. The distal section 225 of the piercing element 220 may cause the porous sample collection media 252 to come into direct contact with a sample receiving area 31 of the assay 30.

The sample collection device 10 and the eluent delivery element 20 may be coupled by bayonet coupling, interference fit, snap fit, or threaded coupling. The eluent delivery element 20 may be constructed to be rotatably inserted into the airflow path 210 (e.g., into the mouthpiece 230). Rotating the eluent delivery element 20 as it is inserted in the airflow path 210 may aid in the piercing of the membrane 23 and may create an opening in the membrane 23 that is larger than the size of the piercing element 220.

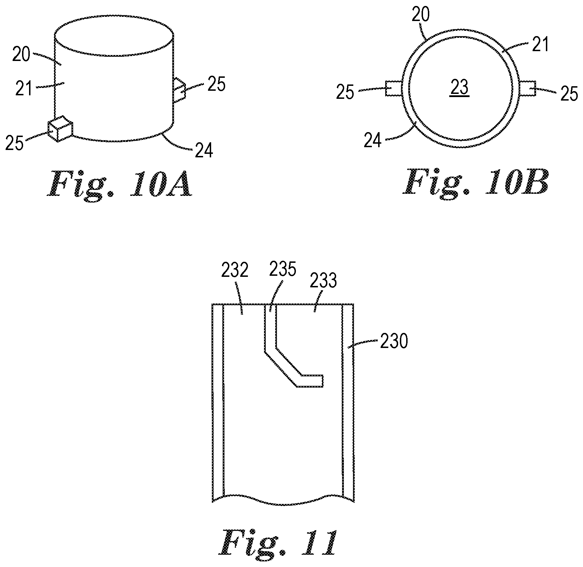

According to an embodiment, the mouthpiece 230 and eluent delivery element 20 include coupling elements to guide the movement of the eluent delivery element 20 as it is received within the interior cavity 232 of the mouthpiece 230. The reservoir 21 may have a cylindrical body and the mouthpiece 230 may have a cylindrical interior cavity 232. The eluent delivery element 20 (e.g., the reservoir 21) is constructed to be at least partially received within the interior cavity 232. The eluent delivery element 20 may include one or more coupling elements 25 (e.g., alignment pins). For example, the eluent delivery element 20 may have two coupling elements 25 (e.g., alignment pins) formed by protrusions extending from opposite sides of the reservoir 21, as shown in FIGURES 10A and 10B. The interior wall 233 of the mouthpiece 230 may include corresponding receiving elements 235 (e.g., grooves), as shown in FIGURE 11 constructed to receive the coupling elements 25 (e.g., alignment pins). The receiving elements 235 may be curved, as shown, to facilitate a bayonet fit, or may be straight (parallel to the longitudinal axis A230).

In some embodiments, the mouthpiece may be constructed of two or more parts to facilitate assembly of the mouthpiece and the insertion of the porous sample collection media. Examples of two-part mouthpieces are shown in FIGURES 12A-16D.

As shown in FIGURES 12A-12D, the mouthpiece 530 may include an inlet part 510 coupled with a base 550. The inlet part 510 defines an air inlet 531 and an opposing distal end 512. The distal end 512 of the inlet part 510 couples with the inlet receiving end 551 of the base 550. The distal end 552 of the base 550 couples with the housing 11 of the sample collection device such that the airflow path 532 aligns with the sample receiving area 31 of the assay 30. Any suitable coupling mechanism (e.g., snap fit, bayonet, interference, treaded, or the like) may be used to couple the base 550 to the housing 11, and the inlet part 510 to the base 550.

The sample collection media 252 may be disposed between the inlet part 510 and the base 550. The base 550 may form a ledge 562 and the distal end 512 of the inlet part 510 may abut the ledge 562. The sample collection media 252 may be disposed between the ledge 562 and the distal end 512 of the inlet part 510. The base 550 and the inlet part 510 may fit together using a snap fit connection that pinches the edges of the sample collection media 252 between the ledge 562 and the distal end 512 of the inlet part 510. For example, the snap fit connection may be formed, in part, by an undercut 553 on the base 550 and by a protrusion (not shown) on the inlet part 510.

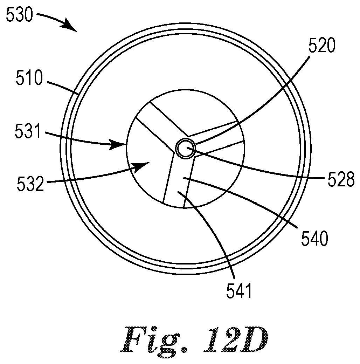

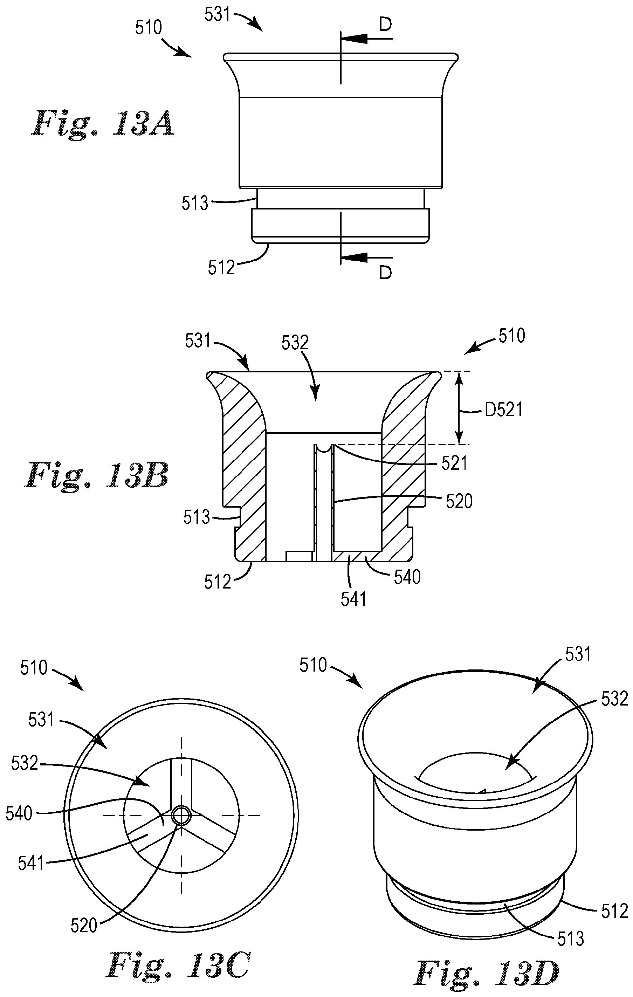

The inlet part 510 (which is shown in more detail in FIGURES 13A-13D) may form a support grid 540. The piercing element 520 may extend from and be supported by the support grid 540. The piercing element 520 may have a longitudinal axis A520 that extends along the longitudinal center axis A530 of the mouthpiece 530. Alternatively, the piercing element 520 may be positioned off-center relative to (not coaxial with) the inlet part 510. The piercing element 520 may have any suitable shape. In some embodiments, such as shown in FIGURES 12C, 12D, 13B, and 13C, the piercing element 520 may be a hollow needle. The piercing element 520 may have a shaft 522 with a hollow center 528. The piercing element may extend from the support grid 540 to a piercing tip 521. The piercing tip 521 is recessed within the inlet part 510. The piercing tip 521 is recessed by a distance D521 from the inlet end of the mouthpiece 530.

The support grid 540 may have any suitable shape. In the exemplary embodiment shown, the support grid 540 has a wheel-and-spoke configuration with a plurality of spokes 541 extending from the center of the support grid 540. The support grid 540 defines opening 544 between the spokes 541.

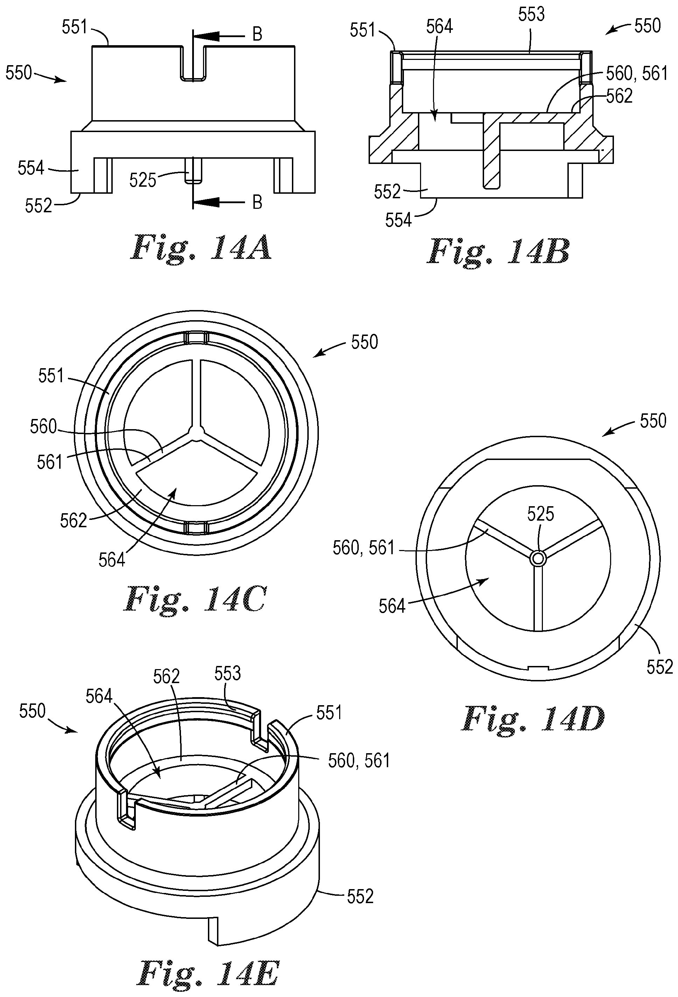

The base 550 (which is shown in more detail in FIGURES 14A-14E) may include a second support grid 560. A distal fluid guide 525 may optionally extend distally (toward the assay) from the second support grid 560. The distal fluid guide 525 may extend along the longitudinal center axis A530 of the mouthpiece 530. The second support grid 560 may also act as a media support for the sample collection media 252. The second support grid 560 may have any suitable shape. In the exemplary embodiment shown, the second support grid 560 has a wheel-and-spoke configuration with a plurality of spokes 561 extending from the center of the support grid 560. The support grid 560 defines opening 564 between the spokes 561.

The interior wall 533 of the mouthpiece 530 may include similar receiving elements 235 (e.g., grooves) as shown in FIGURE 11 constructed to receive the coupling elements 25 of the eluent delivery element 20.

In another embodiment, shown in FIGURES 15A-16D, the support grid 540' of the inlet part 510' has spokes 541' that are angled downward from the interior wall 533 toward the piercing element 520. That is, the spokes 541' may be set at a non-perpendicular angle relative to the longitudinal center axis A520 of the piercing element 520, where the end of the spoke 541' attached to the piercing element 520 is lower (closer to the porous sample collection media 252) than the opposing end. Angling the spokes 541' toward the porous sample collection media 252 may help guide the eluent from the eluent delivery element 20 to the porous sample collection media 252. The rest of the mouthpiece 530' shown in FIGURES 15A-16D may be the same or similar to the mouthpiece 530 shown in FIGURES 12A-14E.

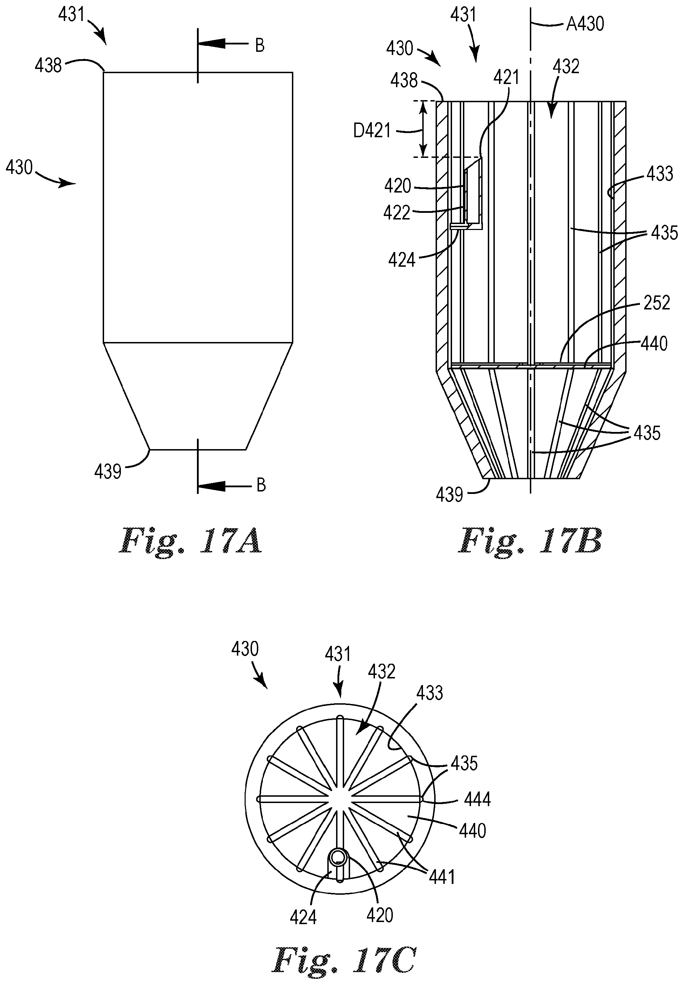

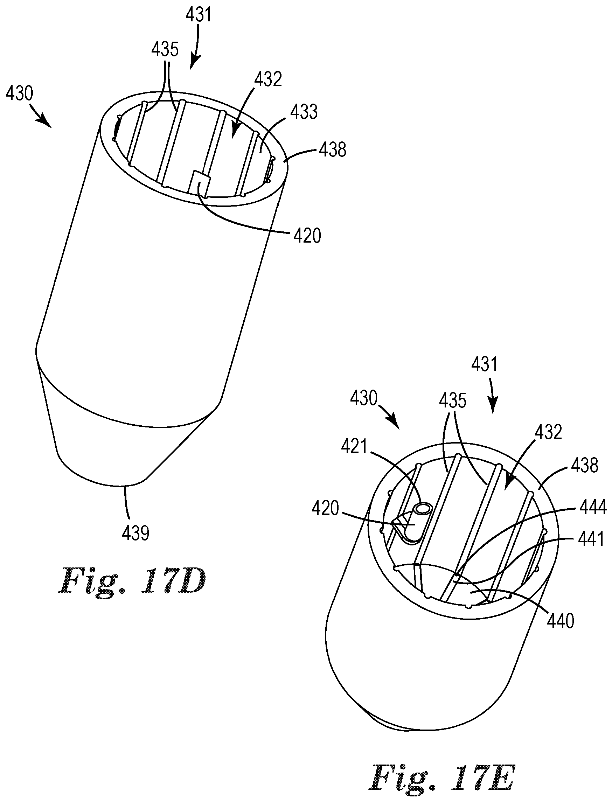

In some embodiments, the piercing element is not co-axial with the mouthpiece. An example of an alternative mouthpiece 430 for use with the sample collection system of FIGURES 1A and 1B is shown in FIGURES 17A-17E. The mouthpiece 430 extends from an inlet end 438 to an outlet end 439 and defines an air inlet 431 at the inlet end 438. The mouthpiece 430 includes a piercing element 420 that is attached to the interior wall 433 of the mouthpiece 430 by an extension 424. The piercing element 420 includes a shaft 422 and a piercing tip 421. The piercing tip 421 is recessed in the interior 432 of the mouthpiece 430 by a distance D421 from the inlet end 438. The extension 424 may position the piercing element 420 in the mouthpiece 430 such that a fluid guide element 20 can be inserted into the mouthpiece 430 and be pierced by the piercing element 420. The piercing element 420 may have any suitable shape, such as the hollow needle shown.

The mouthpiece 430 may include a support grid or platform 440 for supporting the porous sample collection media 252. The platform 440 may be substantially planar and perpendicular to the longitudinal center axis A430 of the mouthpiece. The interior wall 433 of the mouthpiece 430 may include a plurality of grooves or channels 435 that may act as fluid guides. The plurality of channels 435 may extend parallel to the longitudinal axis A430 of the mouthpiece 430. The channels 435 may extend below the platform 440 into the conical lower portion of the mouthpiece 430. The platform 440 may also include wicking channels 441 on the top side of the platform 440. The wicking channels 441 may help guide the eluent from the center of the platform 440 (and sample collection media 252) toward the interior wall 433. The mouthpiece may include a plurality of openings 444 between the platform 440 and the interior wall 433 where the platform 440 meets the channels 435 (see FIGURE 17C).

When the mouthpiece 430 is used, after the eluent delivery element 20 is inserted into the inlet end 438 of the mouthpiece 430, the piercing element 420 pierces the eluent delivery element 20 and eluent may flow from the pierced opening through or along the piercing element 20 and/or the channels 435. The eluent may elute the collected sample from the sample collection media 252 and further be wicked along the wicking channels 441 of the platform 440, through the openings 444, and down the channels 435 below the platform 440, toward the sample receiving area 31 of the assay.

The mouthpiece 430 may be coupled with the housing 11 of the sample collection device by any suitable mechanism, including by using a mechanical coupling (such as bayonet fit, snap fit, pressure fit, interference fit, threaded fit, or the like), an adhesive, or by welding.

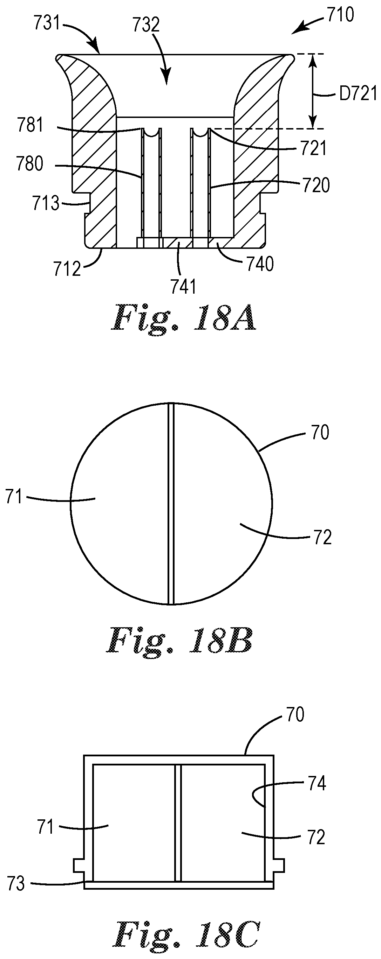

In some embodiments, the eluent delivery element includes a second reservoir containing a fluid, and the mouthpiece includes two piercing elements for the two reservoirs. An example of an inlet part 710 of a mouthpiece with two piercing elements 720, 780 is shown in FIGURE 18A. The first and second piercing elements 720, 780 both extend proximally from the support element 740 to a first piercing tip 721 and a second piercing tip 781, respectively. The tips 721, 781 of the first and second piercing elements 720, 780 are recessed in the mouthpiece (inlet part) 710 by a distance D721, as described above. The first and second piercing elements 720, 780 may be recessed by the same distance or by different distances, as long as both piercing elements 721, 781 are recessed sufficiently to reduce or minimize the likelihood of a user inadvertently coming into contact with the tips 721, 781 of the first and second piercing elements 720, 780. For example, the first and second piercing elements 720, 780 may independently be recessed by 4 mm or more, 5 mm or more, or 6 mm or more. The first and second piercing elements 720, 780 may independently be recessed by a distance of 10 mm or less, 9 mm or less, or 8 mm or less. The inlet part 710 may be otherwise similar to the inlet part 510 shown in FIGURES 13A-13D, including an air inlet 731, an airflow path 732, and a distal end 712. The support element 740 may have any suitable construction and may include spokes 741 or ribs with spaces formed between the spokes 741 or ribs for fluid flow. The inlet part 710 may be used with the base 550 shown in FIGURES 14A-14E to form a mouthpiece. However, the two piercing elements 720, 780 are not limited to the particular shape of the mouthpiece, inlet part 710, or base 550 and can be provided with any mouthpiece or nose piece discussed herein.

The eluent delivery element 70 with two reservoirs 71, 72 is shown in FIGURES 18B and 18C. The eluent delivery element 70 is otherwise similar to the eluent delivery element 20 shown, for example, in FIGURES 10A and 10B, including two coupling elements 75 (e.g., alignment pins) and a membrane 73. The interior of the eluent delivery element 70 is divided by an interior wall 74 into a first reservoir 71 and a second reservoir 72. The first and second reservoirs 71, 72 each include a fluid. For example, the first reservoir 71 may include an eluent (e.g., a metered dose of an eluent) and the second reservoir 72 may include a solution with one or more reagents or other components. The one or more reagents or other components may, for example, participate in elution or transfer of the collected sample or in the reaction or analysis when the collected sample reaches the assay 30. The second reservoir 72 may be sealed by the same membrane 73 as the first reservoir 71, or may be sealed by a second membrane.

When the eluent delivery element 70 is received within the airflow path 732 of the mouthpiece (inlet part) 710, the first and second piercing elements 720, 780 pierce the first and second reservoirs 71, 72. The coupling elements 75 (e.g., alignment pins) may help guide the eluent delivery element 70 into place within the mouthpiece (inlet part) 710. The interior wall of the mouthpiece (inlet part) 710 may include corresponding receiving elements, such as grooves or slots (not shown). The eluent delivery element 70 may be constructed to deliver the fluid from the second reservoir 72 simultaneously with the eluent from the first reservoir 71. The eluent delivery element 70 may be constructed to deliver the fluid from the second reservoir 72 in succession with the eluent from the first reservoir 71.

The housing may include a pre-filter or screen disposed in the airflow path in front (upstream) of the porous sample collection media. The screen may be constructed to catch larger particles (larger than viruses or pathogens) and prevent such particles from reaching the porous sample collection media. The exhalation airflow may passe through a thickness of the pre-filter or screen. The pre-filter or screen may at least partially occlude the air flow path. In some cases, the pre-filter or screen may have a major plane that is orthogonal to the direction of the exhalation airflow passing through the thickness of the pre-filter or screen. The pre-filter or screen may be a non-woven layer configured to filter out larger particles from the exhalation airflow passing through the pre-filter or screen. In some cases, the pre-filter or screen may be a non-woven layer that does not have an electrostatic charge. In some embodiments, the pre-filter or screen does not capture significant amounts of viral material, pathogen material, or other analyte material, and instead allows them to transmit through the pre-filter or screen. In some embodiments, the pre-filter or screen is made of or includes at least one of a plastic mesh, a woven net, a needle-tacked fibrous web, a knitted mesh, an extruded net, and/or a carded or spunbond coverstock. In some embodiments, the pre-filter or screen is part of the support grid or support element structure.

The porous sample collection media may be a nonwoven material capable of capturing pathogens, viruses, or other analytes from an exhalation airflow. According to an embodiment, the porous sample collection media is a nonwoven material carrying an electrostatic charge. The electrostatic charge may enable capturing pathogens, viruses, or other analytes from an exhalation airflow. In some cases, the porous sample collection media may be a hydrophobic nonwoven material. In other cases, the porous sample collection media may be a hydrophilic nonwoven material. The porous sample collection media may be a hydrophobic nonwoven material carrying an electrostatic charge configured to capture pathogens, viruses, or other analytes from an exhalation airflow. The porous sample collection media may be a hydrophilic nonwoven material carrying an electrostatic charge configured to capture pathogens, viruses, or other analytes from an exhalation airflow. The term "hydrophobic" refers to a material having a water contact angle of 90 degrees or greater, or from about 90 degrees to about 170 degrees, or from about 100 degrees to about 150 degrees. The term "hydrophilic" refers to a material having a water contact angle of less than 90 degrees. Water contact angle is measured using ASTM D5727-1997 Standard test method for surface wettability and absorbency of sheeted material using an automated contact angle tester.

The porous sample collection media may be formed of any suitable material that is capable of capturing viruses, pathogens, or other analytes from exhalation airflow and releasing the captured viruses, pathogens, or other analytes upon being contacted with an eluent, such as a saline solution. The porous sample collection media may be formed of polymeric material. The porous sample collection media may be formed of a polyolefin. Examples of suitable polyolefins include polypropylene, polylactic acid, and the like, and a combination thereof. In one embodiment the porous sample collection media is formed of polypropylene. In one embodiment the porous sample collection media is formed of polylactic acid. One illustrative porous sample collection media is commercially available from 3M Company (St. Paul MN, U.S.A.) under the trade designation FILTRETE Smart MPR 1900 Premium Allergen, Bacteria & Virus Air Filter Merv 13.

The porous sample collection media may have a thickness (orthogonal to the major plane) of 200 µm or greater or 250 µm or greater. The porous sample collection media may have a thickness of 750 µm or less or 1000 µm or less. The porous sample collection media may have a thickness of in a range from 200 µm to 1000 µm, or from 250 µm to 750 µm. The porous sample collection media may have major plane surface area (of one side) of 1 cm² or greater or 2 cm² or greater. The porous sample collection media may have major plane surface area of 3 cm² or less or 4 cm² or less. The porous sample collection media may have major plane surface area in a range from 1 cm² to 4 cm², or 2 cm² to 3 cm².

In some embodiments, the porous sample collection media may be pleated. In some embodiments, the pleat frequency is between about 1 pleat per 0.6 cm of media and about 1 pleat per 2 mm of media. In some embodiments, the pleat height is between about 2 mm and about 4 mm. The specific pleating patterns or shape of the pleating features is a generally frustoconical, pleated shape and/or pattern, but any desired pattern and shape may be used.

According to an embodiment, the sample is eluted from the loaded sample collection media by dispensing a liquid (an eluent) onto the loaded sample collection media. The sample may be eluted by a metered dose of the liquid (eluent). The eluent may be an aqueous liquid. The eluent may be a buffer solution. The eluent may be an aqueous buffer solution. The eluent may be a saline solution. The eluent may include a surfactant. The eluent may have a contact angle of greater than 90 degrees when measured on the porous sample collection media. The eluent may be a saline solution including a surfactant. The eluent (e.g., a buffer or a saline solution) may include from 0.1 wt-% or more or 0.5 wt-% or more, and up to 1 wt-% or up to 2 wt-% of surfactant. When provided as a metered dose, the eluent may have a volume of 50 µL or greater, 100 µL or greater, 150 µL or greater, or 200 µL or greater. The metered dose may be 500 µL or less, 400 µL or less, 300 µL or less, or 250 µL or less. The metered dose may be from 50 µL to 500 µL, or from 100 µL to 400 µL. According to an embodiment, the reservoir of the eluent delivery element contains an eluent that may be an aqueous buffer solution or saline solution. According to an embodiment, the reservoir of the eluent delivery element contains a metered dose of the eluent. According to an embodiment, the reservoir of the eluent delivery element contains a volume of eluent that is greater than the metered dose.

The eluent may be applied onto the loaded porous sample collection media. The eluent may be wicked and/or guided by the piercing element. The eluent may travel through the surface and thickness of the loaded porous sample collection media and flow off of the porous sample collection media carrying any virus, pathogen, or other analyte, that was present on the loaded porous sample collection media. This loaded eluent (e.g., eluate) may flow onto the assay and be tested for the presence of a pathogen or other analyte of interest. The eluate may be guided by a fluid flow guide onto the assay.

The assay included in the sample collection system may be any suitable assay. In some embodiments, the assay is a lateral flow assay ("LFA") or a vertical flow assay ("VFA"). LFAs and VFAs are generally paper-based platforms for the detection and quantification of analytes in complex mixtures, including biological samples such as saliva, urine, etc. LFAs and VFAs are typically easy to use and can be used both by professionals in a health care setting or laboratory as well as by lay persons at home. Typically, a liquid sample is placed on the assay in a sample receiving region and is wicked by capillary flow along the device to a test region. LFAs and VFAs are typically based on antigens or antibodies that are immobilized in the test region and that selectively react with the analyte of interest. The result is typically displayed in less than 30 minutes, such as within 5 to 30 minutes. LFAs and VFAs can be tailored for the testing of a variety of viruses and other pathogens, as well as many other types of analytes. According to an embodiment, the assay used in the sample collection system of the present disclosure is constructed for the detection of a target virus, target pathogen, or other target analyte. According to an embodiment, the assay used in the sample collection system of the present disclosure is constructed for the detection of a target virus, target pathogen, or other target analyte, that may be present in the exhalation air flow of a subject.

The housing of the sample collection device may be formed of a rigid material, such as plastic or a paper-based material such as cardboard or cardstock. In some embodiments, the housing is made of plastic. In some embodiments, at least a portion of the housing is transparent. For example, the housing may include transparent material in an area of a result display of the assay. The housing may include a viewing window (either transparent material or an opening) in the area of the result display. In some cases, the entire housing may be made of a transparent material. In other embodiments, the material may be a soft material, such as a closed cell foam.

The sample collection system may be provided as a kit. The kit may include the sample collection device and eluent delivery element and an assay, as discussed above. The kit may include a sample collection device including a housing with an airflow path having a length extending from a proximal end comprising an air inlet to a distal end; a sample collector including porous sample collection media arranged to occlude the airflow path; and a piercing element arranged within the airflow path and extending from the sample collector along the length of the airflow path. The kit may further include an eluent delivery element including a reservoir containing an eluent; and a membrane disposed at a coupling end of the reservoir and sealing the reservoir, where the coupling end is constructed to couple with the proximal end of the airflow path such that the piercing element pierces the membrane. The kit may further include a lateral flow assay arranged or arrangeable to receive a sample from the sample collection element.

A method of obtaining a sample using a sample collection system includes breathing into the proximal end of the airflow path to collect a sample on the porous sample collection media. The exhalation airflow passes through the porous sample collection media, which is constructed to capture viruses, other pathogens, or other analytes, from the exhalation airflow. This results in the formation of a loaded media. The method further includes coupling the coupling end of the eluent delivery element with the first end of the airflow path. Coupling the eluent delivery element with the sample collection device causes the membrane of the eluent delivery element to be pierced, causing eluent to flow from the reservoir onto the loaded media. The method may include transferring a metered dose of the eluent onto the loaded media to elute the collected sample from the porous sample collection media. The eluent elutes the sample from the loaded media. The eluent containing the sample (the eluate) may further flow onto a lateral flow assay. to deposit the collected sample on the lateral flow assay.

The method may further include reading a test result from the lateral flow assay. The result may be read through a viewing window on the housing of the sample collection device.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. It should be understood that this disclosure is not intended to be unduly limited by the illustrative embodiments and examples set forth herein and that such examples and embodiments are presented by way of example only with the scope of the disclosure intended to be limited only by the claims set forth here.

## Claims

1. A sample collection system (1) comprising:
a sample collection device comprising:
a housing (11) comprising an airflow path (210) extending from a proximal end comprising an air inlet (231) to a distal end;
a piercing element (220) arranged within the airflow path; and
a sample collector (250) comprising porous sample collection media (252) arranged to occlude the airflow path; and
an eluent delivery element (20) comprising:
a reservoir (21) containing an eluent (22); and
a membrane (23) disposed at a coupling end (24) of the reservoir and sealing the reservoir,
the coupling end being constructed to couple with the proximal end of the airflow path and be at least partially received within the airflow path such that the piercing element pierces the membrane upon coupling,
wherein the sample collection system has an uncoupled position and a coupled position (P2) where the eluent delivery element is coupled with the sample collection device, and wherein the eluent delivery element is constructed to deliver a metered dose of the eluent from the reservoir onto the porous sample collection media in the coupled position.

2. The sample collection system of claim 1 further comprising a lateral flow assay (30) adjacent the porous sample collection media, optionally wherein the porous sample collection media is in direct contact with the lateral flow assay.

3. The sample collection system of any one of claims 1 to 2, wherein the housing comprises a mouthpiece (230) defining the proximal end of the airflow path, and wherein the piercing element is recessed in the mouthpiece.

4. The sample collection system of any one of claims 1 to 3, wherein the airflow path comprises a longitudinal center axis and wherein the piercing element extends parallel to the longitudinal center axis.

5. The sample collection system of any one of claims 1 to 4, wherein the piercing element comprises a hollow center (228) extending from an open first end to an opposing open second end.

6. The sample collection system of any one of claims 1 to 5, wherein the piercing element comprises surface texture or material configured to facilitate wicking or capillary flow.

7. The sample collection system of any one of claims 2 to 6, wherein the piercing element comprises a distal section (225) in direct contact with a sample receiving area (31) of the lateral flow assay, optionally wherein the distal section comprises surface texture or material configured to facilitate wicking or capillary flow.

8. The sample collection system of any one of claims 1 to 7, wherein the housing comprises a support element (240) in the airflow path and a protrusion (225) extending from the support element in a direction opposite of the piercing element, wherein the protrusion causes the porous sample collection media to be deformed into a cone shape.

9. The sample collection system of any one of claims 2 to 8, wherein the piercing element defines a fluid flow guide (225) extending distally from the sample collector, optionally wherein the fluid flow guide comprises a distal section (226) in direct contact with a sample receiving area of the lateral flow assay, optionally wherein the distal section comprises surface texture or material configured to facilitate wicking or capillary flow.

10. The sample collection system of any one of claims 1 to 9, wherein the sample collection device comprises a mouthpiece (430) defining an interior surface (433), and wherein the interior surface comprises wicking channels (435), optionally wherein mouthpiece comprises a platform (440) and wherein the porous sample collection media is disposed on the platform.

11. The sample collection system of any one of claims 1 to 10, wherein the eluent delivery element comprises a second reservoir (72) containing a fluid, wherein the second reservoir is sealed by a second membrane (73), optionally wherein the piercing element comprises two prongs defining a first piercing tip (721) and a second piercing tip (781), optionally wherein the eluent delivery element is constructed to deliver the fluid from the second reservoir simultaneously with the eluent from the first reservoir.

12. The sample collection system of any one of claims 1 to 11, wherein the porous sample collection media comprises nonwoven material, optionally wherein the nonwoven material comprises polylactic acid, polypropylene, or a combination thereof, optionally wherein the porous sample collection media carries an electrostatic charge.

13. A kit comprising:
the sample collection system of claim 1,
wherein the piercing element extends from the sample collector along the length of the airflow path; and
an assay (30) arranged or arrangeable to receive a sample from the sample collection element.

14. A method of obtaining a sample using the sample collection system (1) of claim 1, the method comprising:
breathing into the proximal end of the airflow path to collect a sample on the porous sample collection media;
coupling the coupling end of the eluent delivery element with the proximal end of the airflow path; and
transferring a metered dose of the eluent from the reservoir onto the porous sample collection media to elute the collected sample from the porous sample collection media, optionally wherein the sample collection system comprises a lateral flow assay (30), and wherein eluting the collected sample causes an eluate comprising the collected sample to be deposited onto the lateral flow assay.

## Patentansprüche

1. Ein Probensammelsystem (1), aufweisend:
eine Probensammelvorrichtung, aufweisend:
ein Gehäuse (11), aufweisend einen Luftstrompfad (210), der sich von einem proximalen Ende, aufweisend einen Lufteinlass (231), zu einem distalen Ende erstreckt;
ein Durchstechelement (220), das innerhalb des Luftstrompfads angeordnet ist; und
einen Probensammler (250), aufweisend poröse Probensammelmedien (252), die angeordnet sind, um den Luftstrompfad zu verschließen; und
ein Eluentabgabeelement (20), aufweisend:
einen Behälter (21), der einen Eluenten (22) enthält; und
eine Membran (23), die an einem Kopplungsende (24) des Behälters angeordnet ist und den Behälter abdichtet,
wobei das Kopplungsende konstruiert ist, um mit dem proximalen Ende des Luftstrompfads zu koppeln und mindestens teilweise innerhalb des Luftstrompfads derart aufgenommen zu werden, dass das Durchstechelement die Membran bei einem Koppeln durchsticht,
wobei das Probensammelsystem eine ungekoppelte Position und eine gekoppelte Position (P2) hat, wobei das Eluentabgabeelement mit der Probensammelvorrichtung gekoppelt ist und wobei das Eluentabgabeelement konstruiert ist, um eine abgemessene Dosis des Eluenten aus dem Behälter auf das poröse Probensammelmedium in der gekoppelten Position abzugeben.

2. Das Probensammelsystem nach Anspruch 1, ferner aufweisend einen Lateral-Fluss-Assay (30), der an das poröse Probensammelmedium angrenzt, gegebenenfalls wobei das poröse Probensammelmedium in direktem Kontakt mit dem Lateral-Fluss-Assay steht.

3. Das Probensammelsystem nach einem der Ansprüche 1 bis 2, wobei das Gehäuse ein Mundstück (230) aufweist, das das proximale Ende des Luftstrompfads definiert, und wobei das Durchstechelement in dem Mundstück versenkt ist.

4. Das Probensammelsystem nach einem der Ansprüche 1 bis 3, wobei der Luftstrompfad eine Längsmittelachse aufweist und wobei sich das Durchstechelement parallel zu der Längsmittelachse erstreckt.

5. Das Probensammelsystem nach einem der Ansprüche 1 bis 4, wobei das Durchstechelement eine hohle Mitte (228) aufweist, die sich von einem offenen ersten Ende zu einem gegenüberliegenden offenen zweiten Ende erstreckt.

6. Das Probensammelsystem nach einem der Ansprüche 1 bis 5, wobei das Durchstechelement eine Oberflächentextur oder ein Material aufweist, die/das konfiguriert ist, um eine Dochtwirkung oder eine Kapillarbewegung zu ermöglichen.

7. Das Probensammelsystem nach einem der Ansprüche 2 bis 6, wobei das Durchstechelement einen distalen Abschnitt (225) in direktem Kontakt mit einem Probenaufnahmebereich (31) des Lateral-Fluss-Assays aufweist, gegebenenfalls wobei der distale Abschnitt die Oberflächentextur oder das Material aufweist, die/das konfiguriert ist, um die Dochtwirkung oder die Kapillarbewegung zu ermöglichen.

8. Das Probensammelsystem nach einem der Ansprüche 1 bis 7, wobei das Gehäuse ein Stützelement (240) in dem Luftstrompfad und einen Vorsprung (225) aufweist, der sich von dem Stützelement in einer dem Durchstechelement entgegengesetzten Richtung erstreckt, wobei der Vorsprung das poröse Probensammelmedium veranlasst, in eine Kegelform verformt zu werden.

9. Das Probensammelsystem nach einem der Ansprüche 2 bis 8, wobei das Durchstechelement eine Fluidstromführung (225) definiert, die sich von dem Probensammler distal erstreckt, gegebenenfalls wobei die Fluidstromführung einen distalen Abschnitt (226) in direktem Kontakt mit einem Probenaufnahmebereich des Lateral-Fluss-Assays aufweist, gegebenenfalls wobei der distale Abschnitt die Oberflächentextur oder das Material aufweist, die/das konfiguriert ist, um die Dochtwirkung oder die Kapillarbewegung zu ermöglichen.

10. Das Probensammelsystem nach einem der Ansprüche 1 bis 9, wobei die Probensammelvorrichtung ein Mundstück (430) aufweist, das eine innere Oberfläche (433) definiert, und wobei die innere Oberfläche Dochtkanäle (435) aufweist, gegebenenfalls wobei das Mundstück eine Plattform (440) aufweist und wobei das poröse Probensammelmedium auf der Plattform angeordnet ist.

11. Das Probensammelsystem nach einem der Ansprüche 1 bis 10, wobei das Eluentabgabeelement einen zweiten Behälter (72) aufweist, der ein Fluid enthält, wobei der zweite Behälter durch eine zweite Membran (73) abgedichtet ist, gegebenenfalls wobei das Durchstechelement zwei Zinken aufweist, die eine erste Durchstechspitze (721) und eine zweite Durchstechspitze (781) definieren, gegebenenfalls wobei das Eluentabgabeelement konstruiert ist, um das Fluid aus dem zweiten Behälter gleichzeitig mit dem Eluenten aus dem ersten Behälter abzugeben.

12. Das Probensammelsystem nach einem der Ansprüche 1 bis 11, wobei das poröse Probensammelmedium Vliesmaterial aufweist, gegebenenfalls wobei das Vliesmaterial Polymilchsäure, Polypropylen oder eine Kombination davon aufweist, gegebenenfalls wobei das poröse Probensammelmedium eine elektrostatische Ladung trägt.

13. Ein Kit, aufweisend:
das Probensammelsystem nach Anspruch 1,
wobei sich das Durchstechelement von dem Probensammler entlang der Länge des Luftstrompfads erstreckt; und
einen Assay (30), der angeordnet ist oder angeordnet werden kann, um eine Probe von dem Probensammelelement aufzunehmen.

14. Ein Verfahren zum Erhalten einer Probe unter Verwendung des Probensammelsystems (1) nach Anspruch 1, das Verfahren aufweisend:
Hineinatmen in das proximale Ende des Luftstrompfads, um eine Probe auf dem porösen Probensammelmedium zu sammeln;
Koppeln des Kopplungsendes des Eluentabgabeelements mit dem proximalen Ende des Luftstrompfads; und
Übertragen einer abgemessenen Dosis des Eluenten aus dem Behälter auf das poröse Probensammelmedium, um die gesammelte Probe von dem porösen Probensammelmedium zu eluieren, gegebenenfalls wobei das Probensammelsystem einen Lateral-Fluss-Assay (30) aufweist und wobei das Eluieren der gesammelten Probe ein Eluat, aufweisend die gesammelte Probe, veranlasst, auf dem Lateral-Fluss-Assay abgelagert zu werden.

## Revendications

1. Système de collecte d'échantillon (1) comprenant :
un dispositif de collecte d'échantillon comprenant :
un boîtier (11) comprenant une voie d'écoulement d'air (210) s'étendant d'une extrémité proximale comprenant une entrée d'air (231) à une extrémité distale ;
un élément de perçage (220) agencé au sein de la voie d'écoulement d'air ; et
un collecteur d'échantillon (250) comprenant un milieu de collecte d'échantillon poreux (252) agencé pour obstruer la voie d'écoulement d'air ; et
un élément d'administration d'éluant (20) comprenant :
un réservoir (21) contenant un éluant (22) ; et
une membrane (23) disposée au niveau d'une extrémité d'accouplement (24) du réservoir et scellant le réservoir,
l'extrémité d'accouplement étant construite pour s'accoupler avec l'extrémité proximale de la voie d'écoulement d'air et être au moins partiellement reçue au sein de la voie d'écoulement d'air de telle sorte que l'élément de perçage perce la membrane lors de l'accouplement,
dans lequel le système de collecte d'échantillon a une position désaccouplée et une position accouplée (P2) où l'élément d'administration d'éluant est accouplé au dispositif de collecte d'échantillon, et dans lequel l'élément d'administration d'éluant est construit pour administrer une dose mesurée de l'éluant à partir du réservoir sur le milieu de collecte d'échantillon poreux dans la position accouplée.

2. Système de collecte d'échantillon selon la revendication 1, comprenant en outre un dispositif de dosage à écoulement latéral (30) adjacent au milieu de collecte d'échantillon poreux, facultativement dans lequel le milieu de collecte d'échantillon poreux est en contact direct avec le dispositif de dosage à écoulement latéral.

3. Système de collecte d'échantillon selon l'une quelconque des revendications 1 à 2, dans lequel le boîtier comprend un embout buccal (230) définissant l'extrémité proximale de la voie d'écoulement d'air, et dans lequel l'élément de perçage est encastré dans l'embout buccal.

4. Système de collecte d'échantillon selon l'une quelconque des revendications 1 à 3, dans lequel la voie d'écoulement d'air comprend un axe central longitudinal et dans lequel l'élément de perçage s'étend parallèlement à l'axe central longitudinal.

5. Système de collecte d'échantillon selon l'une quelconque des revendications 1 à 4, dans lequel l'élément de perçage comprend un centre creux (228) s'étendant d'une première extrémité ouverte à une seconde extrémité ouverte opposée.

6. Système de collecte d'échantillon selon l'une quelconque des revendications 1 à 5, dans lequel l'élément de perçage comprend une texture de surface ou un matériau conçu pour faciliter un effet de mèche ou un écoulement capillaire.

7. Système de collecte d'échantillon selon l'une quelconque des revendications 2 à 6, dans lequel l'élément de perçage comprend une section distale (225) en contact direct avec une zone de réception d'échantillon (31) du dispositif de dosage à écoulement latéral, facultativement dans lequel la section distale comprend une texture de surface ou un matériau conçu pour faciliter un effet de mèche ou un écoulement capillaire.

8. Système de collecte d'échantillon selon l'une quelconque des revendications 1 à 7, dans lequel le boîtier comprend un élément de support (240) dans la voie d'écoulement d'air et une saillie (225) s'étendant à partir de l'élément de support dans une direction opposée à l'élément de perçage, dans lequel la saillie entraîne la déformation du milieu de collecte d'échantillon poreux en forme de cône.

9. Système de collecte d'échantillon selon l'une quelconque des revendications 2 à 8, dans lequel l'élément de perçage définit un guide d'écoulement de fluide (225) s'étendant de manière distale à partir du collecteur d'échantillon, facultativement dans lequel le guide d'écoulement de fluide comprend une section distale (226) en contact direct avec une zone de réception d'échantillon du dispositif de dosage à écoulement latéral, facultativement dans lequel la section distale comprend une texture de surface ou un matériau conçu pour faciliter un effet de mèche ou un écoulement capillaire.

10. Système de collecte d'échantillon selon l'une quelconque des revendications 1 à 9, dans lequel le dispositif de collecte d'échantillon comprend un embout buccal (430) définissant une surface intérieure (433), et dans lequel la surface intérieure comprend des canaux à effet de mèche (435), facultativement dans lequel l'embout buccal comprend une plate-forme (440) et dans lequel le milieu de collecte d'échantillon poreux est disposé sur la plate-forme.

11. Système de collecte d'échantillon selon l'une quelconque des revendications 1 à 10, dans lequel l'élément d'administration d'éluant comprend un second réservoir (72) contenant un fluide, dans lequel le second réservoir est scellé par une seconde membrane (73), facultativement dans lequel l'élément de perçage comprend deux broches définissant une première pointe de perçage (721) et une seconde pointe de perçage (781), facultativement dans lequel l'élément d'administration d'éluant est construit pour administrer le fluide du second réservoir simultanément avec l'éluant du premier réservoir.

12. Système de collecte d'échantillon selon l'une quelconque des revendications 1 à 11, dans lequel le milieu de collecte d'échantillon poreux comprend un matériau non tissé, facultativement dans lequel le matériau non tissé comprend de l'acide polylactique, du polypropylène, ou une combinaison de ceux-ci, facultativement dans lequel le milieu de collecte d'échantillon poreux porte une charge électrostatique.

13. Trousse comprenant :
le système de collecte d'échantillon selon la revendication 1,
dans laquelle l'élément de perçage s'étend à partir du collecteur d'échantillon le long de la longueur de la voie d'écoulement d'air ; et
un dispositif de dosage (30) agencé ou pouvant être agencé pour recevoir un échantillon de l'élément de collecte d'échantillon.

14. Procédé d'obtention d'un échantillon à l'aide du système de collecte d'échantillon (1) selon la revendication 1, le procédé comprenant :
le fait de respirer dans l'extrémité proximale de la voie d'écoulement d'air afin de collecter un échantillon sur le milieu de collecte d'échantillon poreux ;
l'accouplement de l'extrémité d'accouplement de l'élément d'administration d'éluant avec l'extrémité proximale de la voie d'écoulement d'air ; et
le transfert d'une dose mesurée de l'éluant à partir du réservoir sur le milieu de collecte d'échantillon poreux pour éluer l'échantillon collecté à partir du milieu de collecte d'échantillon poreux, dans lequel le système de collecte d'échantillon comprend facultativement un dispositif de dosage à écoulement latéral (30), et dans lequel l'élution de l'échantillon collecté amène un éluat comprenant l'échantillon collecté à être déposé sur le dispositif de dosage à écoulement latéral.
